# FASCICULE DE BREVET EUROPEEN

(11) **EP 1 252 901 B1**
(45) Date de publication et mention de la délivrance du brevet: **24.11.2004**
(21) Numéro de dépôt: 02290970.9
(22) Date de dépôt: 17.04.2002
(51) Int. Cl.: A61L 26/00

(54) **Pansement sous forme pulvérulente et son procédé de fabrication**
Pulverförmiger Wundverband und Verfahren zu seiner Herstellung
Powdered wound dressing and method for producing the same

(30) Priorité: 25.04.2001 FR 0105559
(43) Date de publication de la demande: 30.10.2002
(73) Titulaire: Les Laboratoires Brothier, 92000 Nanterre (FR)
(72) Inventeur: Maingault, Philippe, 49700 Douai la Fontaine (FR)
(74) Mandataire: Bloch, Gérard

(56) Documents cités:
- EP-A- 0 845 271
- WO-A-90/14110
- WO-A-91/11206
- WO-A-92/22285
- WO-A-97/33632

## Description

L'invention concerne un pansement pour les plaies réalisé à base de sels d'alginate ainsi que son procédé de fabrication.

Les alginates sont des substances naturelles qui sont extraites des algues brunes. Certains sels d'alginates sont solubles dans l'eau tels l'alginate de sodium ou de potassium. D'autres sont insolubles tel l'alginate de calcium.

L'utilisation des alginates dans les pansements est en soi bien connue. On les utilise généralement sous la forme d'une masse d'hydrocolloïde ou d'hydrogel ou bien sous une forme fibreuse. Ils sont alors transformés en un produit textile tel qu'un non-tissé fibrillaire, un produit tissé, tricoté ou bien associant les deux.

Le brevet EP 0845271 décrit un tel produit. Le pansement comprend une matrice d'alginate de calcium associée à un ou plusieurs alginates de métal multivalent. Il s'agit d'alginates de zinc, manganèse, cuivre, sélénium ou autres métaux multivalents, à l'exception du magnésium. Ces métaux sont des oligo-éléments, c'est à dire qu'ils sont présents dans le corps humain ou animal en faible quantité et nécessaires à son métabolisme. On fabrique l'alginate de métal multivalent à partir d'alginate de sodium que l'on extrude au moyen d'une filière. Celle-ci est disposée dans un bain de coagulation d'une solution d'un sel soluble du métal multivalent. Pour former le pansement, l'alginate de métal multivalent est associé à la matrice d'alginate de calcium par une technique textile.

La demande de brevet WO-A-91/11206 décrit un matériau alginate contenant des oligo-éléments et qui peut se présenter sous une forme particulaire à appliquer en aérosol.

La présente invention vise à améliorer le traitement des plaies anfractueuses ou cruentées en assurant un meilleur contact entre les tissus des plaies et le pansement. En effet, la forme textile, unitaire, des pansements de l'art antérieur constitue un inconvénient qu'il s'agit de palier.

Conformément à l'invention le pansement d'alginate de calcium se présente sous une forme pulvérulente. Il est caractérisé par le fait qu'il contient au moins l'un des éléments zinc et manganèse en tant qu'oligo-élément et dont la granulométrie est comprise entre 5 et 50µm.

Le pansement conforme à l'invention est ainsi parfaitement biocompatible avec les tissus dans le cas de plaies chroniques et aiguës. Son état divisé permet un contact optimal avec ceux-ci.

Par sa nature le matériau constitue une matrice hydrophile qui adhère aux tissus biologiques et libère au contact des exsudats tissulaires biologiques, les ions qu'elle contient. Ces ions exercent un rôle important dans les mécanismes de la cicatrisation et de l'hémostase. Le produit arrête les saignements et accélère la cicatrisation des tissus biologiques animaux.

Les particules dont le diamètre est inférieur à 5 µm forment des aérosols. Leur diffusion broncho-pulmonaire présente une toxicité respiratoire induite de type emphysème. Par ailleurs, au-delà de 50µm on constate une limitation de la capacité d'écoulement et d'adhérence aux tissus.

Conformément à une autre caractéristique, il comprend entre 8 et 10%, en poids, de calcium, entre 200 et 600 ppm de zinc et entre 20 et 50 ppm de manganèse. De préférence, il comprend environ 9% p/p de calcium, 400ppm de zinc et 30ppm de manganèse.

Conformément à une autre caractéristique, le pansement incorpore entre 200 et 600 ppm d'un additif comme la chlorophylline. Cet additif a une activité antibactérienne.

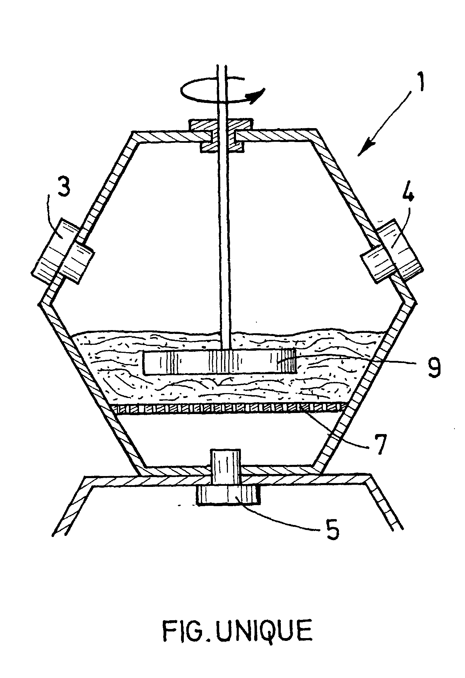

L'invention a également pour objet le procédé de préparation du pansement. Elle sera mieux comprise à la lecture de la description suivante d'un mode de réalisation du procédé de fabrication, en référence à la figure unique annexée, représentant un réacteur.

Le réacteur (1) comprend une enceinte que l'on peut fermer également hermétiquement. Elle est pourvue de trois ouvertures : une première (3) par laquelle on peut charger le réacteur et une deuxième (4) que l'on peut relier à une source de vide et une troisième (5) dans le fond pour évacuer le liquide de filtration. A l'intérieur, on a disposé un support filtrant (7). Un malaxeur à palettes (9) permet d'agiter tout mélange que l'on place sur le support. Pour la mise en oeuvre pratique, on a adapté un dispositif filtre sécheur destiné normalement à la préparation d'extraits végétaux, et fourni par la société Guédu. D'autres dispositifs analogues sont disponibles dans le commerce.

On procède de la façon suivante.

On prépare un mélange d'une poudre d'alginate de sodium que l'on incorpore dans une solution de chlorure de calcium à 250 g/litres dans un rapport d'environ 1,25 (alginate/chlorure) de préférence et se situant dans une plage de 1 à 1,5 et on agite rapidement. On obtient une suspension d'alginate de calcium par échange ionique. On introduit ce mélange dans le réacteur dans lequel on crée un vide. On malaxe le mélange avec rotation lente des palettes 9 avant d'optimiser et homogénéiser l'échange ionique. L'alginate de calcium est produit.

On procède ensuite au lavage de l'alginate de calcium dans le but d'éliminer les ions calcium et chlorure en excès. On introduit de l'eau purifiée sous vide puis on extrait les rejets de filtration par l'orifice de purge (5) en introduisant de l'air filtré sous pression, par l'ouverture (4). Selon l'analyse des rejets, on peut procéder à plusieurs lavages successifs.

Ensuite on sèche partiellement l'alginate de calcium par évaporation de l'eau en créant un vide. La dépression est maintenue entre -0,985 bar et -0,998 bar. Tout en malaxant, on chauffe modérément à une température comprise entre 40 et 55°C pour ne pas dégrader le produit. L'apport calorifique est fourni par la circulation d'un fluide caloporteur dans une double enveloppe de l'enceinte. Le fluide est de préférence l'eau ou la vapeur. Le vide permet de limiter le niveau de température. On obtient une poudre humide ou pâte sèche. On mesure sa teneur en eau par thermogravimétrie, et on en déduit la masse d'alginate. Par calcul, on détermine la quantité d'oligo-éléments à ajouter pour obtenir la concentration finale requise dans la masse d'alginate.

Par exemple, pour une masse sèche calculée de 100 kg d'alginate, on incorpore quelques grammes d'oligo-éléments (40 g de zinc gluconate, 3 g de manganèse gluconate).

Ces oligoéléments sont mis en solution dans un volume d'environ 100 litres pour 100 kg d'alginate (dans une plage de 75 litres à 125 litres).

Séparément on prépare la solution d'oligo-éléments, zinc et manganèse, à laquelle on ajoute le cas échéant un additif tel que la chlorophylline. On dose les éléments en fonction de la quantité que l'on souhaite obtenir dans le produit final. Selon un mode préféré de réalisation on dose les éléments à 400 ppm en zinc et 30ppm en manganèse.

L'incorporation de chlorophylline à ce stade du procédé permet son adsorption dans la masse d'alginate. On ajoute 40 g dans 100 litres d'eau. Par comparaison, l'incorporation de chlorophylline est plus complexe dans un pansement d'alginate de calcium qui se présente sous une forme textile. Il faut, pour ce dernier, mettre en oeuvre une technique de teinture textile.

On transfère les solutions sous vide et on malaxe le mélange sous vide. Cette étape permet d'éviter toute perte de solution et donc d'écart en éléments rajoutés.

On sèche de nouveau le mélange à une température comprise entre 40 et 55°C, tout en mettant l'enceinte sous vide. La dépression est maintenue entre -0,985 et -0,998 bar. On continue à malaxer pendant le séchage pour éviter la formation d'agglomérats.

Enfin, on procède au broyage et au tamisage du produit en sélectionnant la poudre pour que la granulométrie soit comprise entre 5 et 50 µm.

On note que le mélange en milieu humide garantit une homogénéité du produit final que l'on ne pourrait obtenir si l'on procédait à sec.

## Revendications

1. Pansement d'alginate de calcium se présentant sous une forme pulvérulente, **caractérisé par le fait qu'**il contient au moins l'un des éléments zinc et manganèse en tant qu'oligo-élément et dans lequel la granulométrie de la poudre est comprise entre 5 et 50 µm.

2. Pansement selon la revendication précédente, comprenant entre 8 et 10 %, en poids, de calcium, entre 200 et 600 ppm de zinc et entre 20 et 50 ppm de manganèse.

3. Pansement selon l'une des revendications précédentes, comprenant en outre entre 200 et 600 ppm d'un additif tel que la chlorophylline.

4. Procédé de fabrication d'un pansement selon l'une des revendications précédentes, **caractérisé par le fait qu'**il comprend les étapes suivantes :
préparation d'une solution de chlorure de calcium,
incorporation de la poudre d'alginate de sodium dans la solution chlorure de calcium pour obtenir une suspension d'alginate de calcium, par échange ionique,
lavage de la suspension solide, jusqu'à élimination de l'excès des ions calcium et chlorure,
séchage partiel de la suspension
incorporation d'une solution contenant les dits oligo-éléments
séchage et broyage du produit jusqu'à obtention de la granulométrie requise.

5. Procédé selon la revendication précédente, dans lequel, après incorporation de l'alginate de sodium, on procède au mélange sous vide de la solution afin de favoriser l'échange ionique.

6. Procédé selon l'une des revendications 4 et 5, dans lequel le fait que le séchage partiel est opéré à une température comprise entre 40 et 55°C et sous vide à une dépression comprise entre -0,985 bar et -0,998 bar.

7. Procédé selon la revendication 4, 5 ou 6, dans lequel ledit séchage partiel est effectué jusqu'à obtenir une poudre humide.

8. Procédé selon l'une des revendications précédentes, dans lequel on mélange ladite solution contenant les oligo-éléments avec l'alginate par malaxage sous vide.

9. Procédé selon la revendication 4, selon lequel la solution contenant les oligo-éléments contient aussi un additif tel que la chlorophylline.

## Patentansprüche

1. Pulverförmiger Calciumalginat-Verband, **dadurch gekennzeichnet, daß** er wenigstens eines der Elemente Zink und Mangan als Spurenelement enthält und wobei die Granulometrie des Pulvers zwischen 5 und 50 µm liegt.

2. Verband nach dem vorherigen Anspruch enthaltend zwischen 8 bis 10 Gew.-% Calcium, zwischen 200 und 600 ppm Zink und zwischen 20 und 50 ppm Mangan.

3. Verfahren nach einem der vorherigen Ansprüche enthaltend außerdem 200 bis 600 ppm eines Additivs wie Chlorophylline.

4. Verfahren zur Herstellung eines Verbandes nach einem der vorherigen Ansprüche, **dadurch gekennzeichnet, daß** es die Schritte aufweist:
Präparieren einer Calciumchloridlösung,
Einbringen von Natriumalginatpulver in die Calciumchloridlösung, um eine Calciumalginat-Suspension durch Ionenaustausch zu erzielen,
Spülen der festen Suspension bis zur Entfernung der überschüssigen Calcium- und Chlorionen,
partielles Trocknen der Suspension,
Einbringen einer die genannten Spurenelemente enthaltenden Lösung,
Trocknen und Zerkleinern des Produktes bis zur Erzielung der erforderlichen Granulometrie.

5. Verfahren nach dem vorherigen Anspruch, wobei nach dem Einbringen des Natriumalginats das Gemisch im Vakuum weiterverarbeitet wird, um den Ionenaustausch zu fördern.

6. Verfahren nach einem der Ansprüche 4 und 5, wobei das partielle Trocknen bei einer Temperatur zwischen 40 und 55°C und im Vakuum bei einem Unterdruck zwischen -0,985 bar und -0,998 bar erfolgt.

7. Verfahren nach Anspruch 4, 5 oder 6, wobei das genannte teilweise Trocknen bewirkt wird, bis ein feuchtes Pulver erzielt wird.

8. Verfahren nach einem der vorherigen Ansprüche, wobei man die die Spurenelemente enthaltende Lösung mit dem Alginat durch Mischen im Vakuum mischt.

9. Verfahren nach Anspruch 4, wobei die die Spurenelemente enthaltende Lösung auch ein Additiv wie Chlorophylline enthält.

## Claims

1. Wound dressing formed from calcium alginate presented in powder form, **characterised in that** it contains at least one of the elements zinc and manganese as trace elements, and wherein the grain size distribution of the powder is between 5 and 50 µm.

2. Wound dressing according to the preceding claim, comprising between 8 and 10 % by weight calcium, between 200 and 600 ppm zinc and between 20 and 50 ppm manganese.

3. Wound dressing according to one of the preceding claims, comprising in addition between 200 and 600 ppm of an additive such as chlorophylline.

4. Method of manufacturing a wound dressing according to one of the preceding claims, **characterised in that** it comprises the following steps:
preparation of a solution of calcium chloride,
incorporation of the sodium alginate powder in the calcium chloride solution in order to obtain a suspension of calcium alginate, by ion exchange,
washing of the solid suspension until the excess of calcium and chloride ions is eliminated,
partial drying of the suspension,
incorporation of a solution containing said trace elements,
drying and grinding of the product until the required grain size distribution is obtained.

5. Method according to the preceding claim, wherein, after incorporation of the sodium alginate, one proceeds to mixing of the solution under vacuum in order to encourage the ion exchange.

6. Method according to one of claims 4 and 5, wherein the partial drying is operated at a temperature of between 40 and 55°C and under vacuum at a negative pressure of between -0.985 bar and -0.998 bar.

7. Method according to claim 4, 5 or 6, wherein said partial drying is carried out until a moist powder is obtained.

8. Method according to one of the preceding claims, wherein said solution containing the trace elements is mixed with the alginate by blending under vacuum.

9. Method according to claim 4, according to which the solution containing the trace elements also contains an additive such as chlorophylline.
